# EUROPÄISCHE PATENTSCHRIFT

(11) **EP 1 109 519 B1**
(45) Veröffentlichungstag und Bekanntmachung des Hinweises auf die Patenterteilung: **06.10.2004**
(21) Anmeldenummer: 99952435.8
(22) Anmeldetag: 17.08.1999
(51) Int. Cl.: A61F 9/007

(54) **GLAUKOMSTENT**
INTRA-OCCULAR IMPLANT TO ASSIST DRAINAGE OF THE AQUEOUS HUMOUR
IMPLANT INTRA-OCCULAIRE POUR FAVORISER L'EVACUATION DE L'HUMEUR ACQUEUSE

(30) Priorität: 02.09.1998 DE 19840047
(43) Veröffentlichungstag der Anmeldung: 27.06.2001
(73) Patentinhaber: Neuhann, Thomas, 80801 München (DE)
(72) Erfinder: Neuhann, Thomas, 80801 München (DE)
(74) Vertreter: Rösler, Uwe, Dipl.-Phys.
(86) Internationale Anmeldenummer: PCT/DE1999/002588
(87) Internationale Veröffentlichungsnummer: WO 2000/013627

(56) Entgegenhaltungen:
- EP-A- 0 550 791
- EP-A- 0 898 947
- WO-A-80/01460
- WO-A-83/00420
- WO-A-95/08310
- US-A- 4 936 825
- US-A- 5 180 362
- DATABASE WPI Section PQ, Week 198142 Derwent Publications Ltd., London, GB; Class P32, AN 1981-K9310D XP002128098 -& SU 799 746 A (OMSK MED INST), 30. Januar 1981 (1981-01-30)

## Beschreibung

### Technisches Gebiet

Die Erfindung bezieht sich auf eine Vorrichtung zur gezielten Verbesserung und/oder dauerhaften Gewährleistung des Durchlässigkeitsvermögens für Augenkammerwasser durch das Trabekelwerk in den Schlemm'schen Kanal.

### Stand der Technik

In Fällen, in denen das Augenkammerwasser nicht in ausreichendem Maße aus dem Augeninneren entweichen kann, nimmt der Augeninnendruck zu, wodurch sich die Gefahr der Ausbildung eines Glaukom, zu deutsch, grüner Star, erhöht. Der grüne Star ist eine besondere Form der Sehnervenatrophie, welche überwiegend durch einen, für seine gesunde Funktionsfähigkeit zu hohen Augeninnendruck zustandekommt.

Konsequenterweise ist die Senkung des Augeninnendruckes auf wenigstens Werte im statistischen Normbereich erstes Ziel aller therapeutischen Bemühungen bei der Glaukombehandlung.

Die Augendrucksenkung kann durch eine Reihe von Medikamenten, durch Laserverfahren der sogenannten Argon-Laser-Trabekuloplastik (ALT) oder durch operative Eingriffe im engeren Sinne erreicht werden.

Nachteile der medikamentösen Therapie sind ein nur begrenztes Drucksenkungspotential, unerwünschte Nebenwirkungen unterschiedlicher Art je nach verwendeter Substanz und vor allem die Notwendigkeit der lebenslangen mehrfach täglichen Anwendung, die naturgemäß mit Problemen der verläßlichen Anwendung (Compliance) verbunden ist.

Nachteile der Argon-Laser-Trabekuloplastik sind ihr begrenztes Drucksenkungspotential und ihre nur vorübergehende Wirkung durch Nachlassen des Effektes über die Zeit.

Unter den operativen Verfahren sind die sog. fistulierenden Eingriffe heutiger operativer Standard. Unter ihren zahlreichen Nachteilen seien besonders hervorgehoben das erhebliche Potential an postoperativen Komplikationen, die beschleunigte Entwicklung einer Linsentrübung , die Unvorhersagbarkeit des Effektes der von übermäßiger Drucksenkung bis zur raschen Vemarbung mit völligem Verlust der drucksenkenden Wirkung reichen kann.

Diese insgesamt unbefriedigende Erfolgsbilanz und Erfolgsvorhersagbarkeit dieses Standardeingriffes hat zu zahlreichen anderen operativen Ansätzen geführt, von denen insbesondere die Trabekulotomie und die tiefe Sklerektomie genannt seien, beides Operationsmethoden, die einen erleichterten Kammerwasserabfluß durch das Vorsehen physiologischer Abflußwege ermöglichen. Auch diesen Verfahren ist als Nachteil jedoch zu eigen, daß ihr Effekt teilweise oder ganz durch Wundheilungsvorgänge wieder verloren gehen kann.

Die nachfolgenden Ausführungen sollen dem besseren Verständnis der Glaukom-Problematik dienen:

Der Raum zwischen der Augenlinse und der Hornhautrückfläche, die durch die Regenbogenhaut, der Iris, in die hintere und die vordere Augenkammer unterteilt wird, ist von Kammerwasser angefüllt. Das Kammerwasser wird fortlaufend vom Strahlenkörper, dem Ciliarkörper des Auges gebildet und in die hintere Augenkammer abgegeben. Von dort fließt das Kammerwasser durch die Pupille in die vordere Augenkammer, wo es einer Wärmeströmung unterliegt und gelangt von dort in den Kammerwinkel durch das Maschenwerk des corneoskleralen Trabekelwerkes in den Schlemm'schen Kanal. Von hier aus gelangt das Kammerwasser schließlich durch Abflußkanäle in das Venensystem der Augenoberfläche.

Die Aufgabe der in einem homöostatischen Gleichgewicht stehenden Kammerwassersekretion und seines Abflusses ist die Aufrechterhaltung eines in engen Grenzen konstanten Augeninnendruckes, welcher hoch genug sein muß, um die Formstabilität des Auges aufrechtzuerhalten, jedoch niedrig genug, um die Ernährung des Sehnerven nicht zu behindern. Als Normbereich des Augeninnendruckes gelten grob Werte von 10 mm Quecksilbersäule bis 20 mm Quecksilbersäule. Eine scharfe Abgrenzung zwischen normalen und krankhaft erhöhten Werten gibt es jedoch nicht: Der Übergang ist fließend, wobei mit zunehmender Höhe der Augeninnendruckwerte eine Glaukomerkrankung immer wahrscheinlicher wird.

Eine krankhafte Erhöhung des Augeninnendruckes kann prinzipiell sowohl durch übermäßige Kammerwassersekretion wie durch zu geringen Abfluß verursacht werden. Für die Zwecke dieser Darstellung soll nur die dem sog. primären chronischen Offenwinkelglaukom (Glaucoma chronicum simplex) zugrundeliegende Erhöhung des Abflußwiderstandes im juxta-canaliculären Trabekelwerk eingegangen werden, die nach der wissenschaftlichen Literatur für etwa 85-90% aller dieser Glaukome verantwortlich ist.

Das Glaukoma chronicum simplex stellt seinerseits mehr als ¾ aller Glaukomfälle dar. Die Ursachen für diese Widerstandserhöhung im juxta-canaliculären Trabekelwerk sind im einzelnen nicht letztlich geklärt. Genetische Faktoren, die Anlagerung von Substanzen an das Maschenwerk, die die Maschenweite verengen und damit den Widerstand erhöhen sowie ein mechanischer Kollaps des Maschenwerkes sind Aspekte die als gesichert gelten.

Da heute verwendete Prinzip der Trabekulotomie stellt sich so dar:

Ausgehend von der an sich bekannten Trabekulotomie wird nun der Schlemm'sche Kanal von außen aufgesucht und geöffnet. Anschließend wird eine Metallsonde in den Kanal eingeführt und in die Vorderkammer eingeschwenkt. Hierdurch wird jedoch das gesamte Trabekelwerk regelrecht zerrissen, wodurch eine offene Verbindung zwischen der vorderen Augenkammer und dem darin zirkulierenden Kammerwasser und dem Schlemm'schen Kanal hergestellt wird.

Die anatomische Struktur des Trabekelwerks, in dem der erhöhte Abfußwiderstand liegt, wird mit der beschriebenen Vorgehensweise jedoch regelrecht zerstört. Obwohl diese Operationsmethode anfänglich nur wenig überzeugende Resultate lieferte, hat sie in den letzten Jahren durch verfeinerte Ausführungen erheblich an Bedeutung gewonnen. So sind unter bestimmten Bedingungen die Drucksenkungserfolge mit dieser Methode von keiner anderen Methode erreicht worden. Ihr Problem besteht jedoch darin, daß sich die beiden Enden der Aufriß-Strecke wieder verschließen können, so daß nur die tatsächlich aufgerissene Strecke, nicht jedoch der gesamte Kanalumfang für den Abfluß zur Verfügung steht.

Hinzukommt, daß das aufgerissene Trabekelwerk in bestimmten Fällen wieder verkleben kann, indem sich die beiden aufgerissenen Teile des Trabekeiwerkes gleichsam türflügelartig wieder schließen. Solche Wieder-Verschlüsse werden durch Rückflußblutungen aus dem mit dem Schlemm'schen Kanal verbundenen Venensystem gefördert.

So geht aus der EP 0 550 791 A1 ein chirurgisches Operationsinstrument hervor, das zur gezielten Injektion eines hochviskosen Mediums in das Trabekelwerk speziell ausgebildet ist. Insbesondere handelt es sich hierbei um eine Operationssonde, die während der Operation in den Schlemm'schen Kanal eingeführt wird und nach der Behandlung aus diesem vollständig wieder entfernt wird.

Die bogenförmig ausgebildete Sonde weist an ihrer Bogeninnenseite Öffnungen auf, durch die das hochviskose Medium in das Trabekelwerk injiziert wird. Hierzu ist die bogenförmige Sonde mit einem Injektionsgerät verbunden und wird in den Schlemm'schen Kanal eingeführt. Eine detaillierte Darstellung der Funktionsweise des Injektionsvorganges geht aus Fig. 4 hervor.

Bei der bekannten Vorrichtung handelt es sich um ein Operationsinstrument zur Durchführung einer chirurgischen Maßnahme am Auge, um durch gezielte Injektion in das Trabekelwerk dieses lokal aufzuweiten, um dessen Durchlässigkeit für das Kammerwasser zu verbessern.

Prinzipiell würde eine vergleichsweise kleine Öffnung im Trabekelwerk genügen, um den gewünschten Effekt der Augeninnendruck-Regelung zu ermöglichen, wenn denn gewährleistet werden könnte, daß die Öffnung nicht verschlossen wird, so daß das Kammerwasser durch diese wenn auch kleine Lücke Zugang zum gesamten Schlemm'schen Kanal und damit zum natürlichen Abflußsystem findet. Nach Behandlung des Trabekelwerks mit den bislang bekannten Maßnahmen kann jedoch nicht verhindert werden, daß sich künstliche Perforationen oder Aufweitungen im Trabekelwerk durch natürliche Ablagerungen wieder schließen, wodurch die Hauptursache für das Auftreten von Glaukom langfristig nicht beseitigt zu sein scheint.

Aus einer Vielzahl von Druckschriften gehen darüber hinaus Maßnahmen zur Glaukombehandlung hervor, die durch Einführen mechanischer Mittel in den Schlemm'schen Kanal eine lokale Dillatation des Schlemm'schen Kanals hervorrufen und zugleich eine mechanische Deformation des unmittelbar an den Schlemm'schen Kanal angrenzenden Trabekelwerkes bewirken, ohne das Trabekelwerk vor oder nach Einführen des Mittels chirurgisch zu behandeln. Durch die gezielt ausgeübte mechanische Deformation des Trabekelwerkes ist in den meisten Fällen eine Erniedrigung des Abflußwiderstandes für das Kammerwasser aus dem Inneren des Auges durch das Trabekelwerk hindurch zu verzeichnen.

So geht aus der WO 95/08310 ein zumindest teilweise in den Schlemm'schen Kanal implantierbarer Drainagekanal hervor, dessen Kanalwand mit Löchern durchsetzt ist, durch die Kammerflüssigkeit aus dem angrenzenden Trabekelwerk in das Innere des Drainagekanals gelangen kann, durch den die Kammerflüssigkeit entsprechend abgeführt werden kann.

In der US 4,936,825 wird mit Hilfe eines Ringkanals vorgeschlagen Kammerwasser aus der vorderen Augenkammer extrakorporal zu verbringen. Hierzu durchragt der Ringkanal, der seinerseits an seiner Ringkanalwand eine Vielzahl kleiner Öffnungen aufweist, den Bereich der Hornhaut von Außen in das Innere der vorderen Augenkammer. Durch die Öffnungen gelangt das Kammerwasser in den Ringkanal, der das überschüssige Kammerwasser an die äußere Oberfläche der Hornhaut verbringt.

Zum Einbringen einer gezielten Injektion eines hochviskosen Mediums in das Trabekelwerk wird in der SU 799746 eine Mikrokanüle in das Innere des Schlemm'schen Kanals eingeführt, vergleichbar der bereist in der vorstehend zitierten EP 0 550 791 A1.

Aus der WO 83/00420 geht eine implantateinheit hervor, die zwischen die Hornhaut und dem Bereich der iris auf chirurgische Weise implantiert wird und die dafür sorgt, dass das Kammerwasser der vorderen Augenkammer direkt in den Bereich gelangt, in dem das Kammerwasser über natürliche Abflußkanäle in das Venensystem der Augoberfläche gelangen kann.

In der US 5,180 362 wird eine Operationsmethode am Auge beschrieben, bei der eine Operationskanüle quer durch die gesamte vordere Augenkammer geschoben wird, um von der Innenseite des Auges im Bereich des Kammerwinkels einen mechanischen Abflußkanal durch das Trabekelwerk hindurch nach Außen in den Bereich des Schlemm'schen Kanals zu schaffen. Um zu gewährleisten, dass der mechanisch durch die Operationskanüle hervorgerufene Abflußkanal nach Entfernen der Kanüle erhalten bleibt und sich durch Wundheilprozesse nicht wieder verschließt, wird eine spiralartig geformte Kanüle im Inneren des Abflußkanal implantiert, die auch nach Entfernen der Operationskanlüe im Auge verbleibt.

In vorteilhafter Ergänzung zu der in der eingangs zitierten EP 0 550 791 A1 ist in der EP 0898 947 A2 eine Vorrichtung zum Verbessern des Kammerwasserabflusses in einem Auge beschrieben, die ein Stützelement vorsieht, das innerhalb des Schlemm'schen Kanals implantierbar ist und eine andauernd dillatierende Wirkung auf das an den Schlemm'schen Kanal angrenzende Trabekelwerk ausübt, wodurch der Abflußwiderstand für das Kammerwasser reduziert wird. Öffnungen in der Wandung des Stützelement sorgen dafür, dass Kammerwasser aus dem Trabekelwerk in das Stützelement abfließen und in das Sklerabecken gelangen kann, wo es schließlich dem Venensystem zugeführt wird.

### Darstellung der Erfindung

Der Erfindung liegt die Aufgabe zugrunde die vorstehend geschilderte Problematik bei der Durchführung von Operationen zur Behebung von Glaukom und insbesondere zur Wiederherstellung einer gezielten Regulierung des Augeninnendruckes dahingehend zu lösen, daß ein Wiederverschluß von, in das Trabekelwerk eingebrachter Durchführungskanälen vollständig vermieden werden soll. Insbesondere sollen die traumatischen Gewebeirritationen innerhalb des Trabekelwerkes reduziert werden, wodurch auch der Wundheilungsprozeß verbessert werden kann.

Die Lösung der Erfindung zugrundeliegenden Aufgabe ist Gegenstand des Anspruchs 1. Vorteilhafte Weiterbildungen sind Gegenstand der Unteransprüche.

Erfindungsgemäß ist eine Vorrichtung zur gezielten Verbesserung und/oder dauerhaften Gewährleistung des Durchlässigkeitsvermögens für Augenkam nerwasser durch das Trabekelwerk in den Schlemm'schen Kanal, mit einem röhrchenartig ausgebildeten Element, wie in Anspruch 1 definiert.

So wird in den Schlemm'schen Kanal ein Röhrchen, ein sogenannter Stent, eingeführt. Die Aufgabe dieses Stents ist es zum einem das Trabekelwerk an seiner Innenseite aufzuspreizen und somit den Abflußwiderstand zu erniedrigen. Genügt diese Abflüßwiderstandserniedrigung nicht aus, ermöglicht der Stent zum anderen das Trabekelwerk an seiner durchlässigen, dem Trabekelwerk zugewandten Seite zu eröffnen oder zu entfernen, und dabei den Wiederverschluß der Öffnung im Trabekelwerk und das Verkleben des Schlemm Kanals zu verhindern.

Das Kammerwasser erhält somit ungehindert Zugang zur gesamten Circumferenz des Schlemm Kanals und seinen abführenden Kanälen.

Der Stent ist aus einem Gewebeverträglichen Material gearbeitet und verbleibt auch nach der Operation innerhalb des Schlemm'schen Kanals, um diesen dauerhaft zu dillatieren.

Um diesen Anforderungen gerecht zu werden muß das Röhrchen oder Stent aus geeignetem Material gefertigt sein, welches eine äußere Form besitzt, die weitgehend inneren Form des Schlemm'schen Kanals entspricht oder diesen neu formt. Überdies weist das Röhrchen im Inneren einen Hohlkanal auf, der gerade genügend Wandstärke übrigläßt, um einen Kollaps des Stents zuverlässig zu verhindern.

Das Röhrchen ist grundsätzlich von beliebiger Länge, sinnvollerweise entspricht die Länge des Röhrchens nicht weniger als 30° und nicht mehr als 90° im Bogenmaß des ringförmig verlaufenden Schlemm'schen Kanals. Andere Längen und ihre sinnvolle Anwendung sind jedoch grundsätzlich denkbar.

Das Röhrchen besitzt eine Krümmung, die der Krümmung des Schlemm'schen Kanals im zu operierenden Auge besitzt. So ist das Röhrchen entweder individuell anzufertigen oder aber zu standardisieren.

Das Röhrchen weist auf der konkaven Seite wenigstens eine Öffnung im Wandmaterial auf. Das Röhrchen kann auch aus einem Wandmaterial gefertigt sein, das selbst durchlässig ist. So eignen sich hierzu beispielsweise Materialgeflechte mit einer endlich großen bzw. kleine Maschenweite durch die das Kammerwasser hindurchfließen kann. Beispielsweise eignen sich Geflechte, die zum einen die Wasserdurchlässigkeit aufweisen und überdies die für die genannten Dehnungen erforderliche Steifigkeit und Widerstandsfähigkeit besitzen.

Außerdem ist das Röhrchen an seinen beiden Enden geöffnet. Alle Öffnungskanten sind feinstpoliert und abgerundet. Das verwendete Material muß rigide genug sein, um den Kollaps des Kanals zu verhindern und völlig gewebeverträglich sein, um Wundheilungsvorgänge zu verhindern. Eine Beispielsausführung könnte aus Titan, galvanisch vergoldetem Implantationsstahl oder anderen Materialien sein.

Eine bevorzugte Weiterbildung des Stents sieht, neben wenigstens einer Öffnung, die dem Trabekelwerk zugewandt ist und die sowohl als Ablußöffnung für das Kammerwasser dient, als auch als Durchgangsöffnung für ein mikrochirurgisches Instrument dienen kann, das längs in den Stent eingeführt und dessen distales Ende seitlich durch die Durchgangsöffnung innerhalb des Stents geführt werden kann, um in das Trabekelwerk hineinzuragen, um dort mechanisch kleinste Durchbrüche im Trabekelwerk zu erzeugen, wenigstens eine weitere, kleinere Öffnung, vorzugsweise zwei Öffnungen, an der dem Trabekelwerk abgewandten Seite des Stents vor. Die, im Durchmesser kleiner, als im Vergleich zur Abflußöffnung bemessenen Öffnungen sollen hauptsächlich einer besseren Handhabbarkeit des Stents, während seines Einbringens in den Schlemm'schen Kanals dienen. Hierzu sind zwei Öffnungen im Stent nebeneinander und unmittelbar der Abflußöffnung gegenüberliegend angeordnet. Durch die kleinen Öffnungen kann ein Haltedraht von der Rückseite des Stents geführt werden, wobei sich innerhalb des Stents zwischen den kleinen Öffnungen eine Schlinge bildet. Beide lose Enden des Haltedrahtes ragen zur Rückseite des Stents aus den Öffnungen heraus und können von einem Halte- und Betätigungsinstrument erfaßt werden. Die Aufnahme des Stents mit dem Halte- und Betätigungsinstrument erfolgt derart, daß der Stent sicher und fest mit diesem verbunden ist. Auf diese Weise läßt sich nun der Stent in den Schlemm'schen Kanal, durch eine entsprechende Öffnung leicht einsetzen.

Nach Einbringen des Stents in den Schlemm'schen Kanal kann der Draht von dem Halte- und Betätigungsinstrument gelöst und vollständig von dem Stent entfernt werden, indem der Draht lediglich mit einem losen Ende durch die beiden kleinen Öffnungen ausgefädelt wird. Der, in den Stent eingebrachte Haltedraht dient jedoch, neben seiner Haltefunktion auch einem verbesserten Perforieren bzw. lokalen Durchschneiden des Trabekelwerkes selbst. Hierzu wird der Haltedraht von seiner festen Verbindung zum Halte- und Betätigungsinstrument etwas gelöst, so daß die Drahtschlinge, die sich innerhalb des Stents zwischen beiden kleinen Öffnungen bildet, in das Innere des, der Abflußöffnung gegenüberbefindliche Trabekelwerks geschoben werden kann, um hier das Gewebe lokal zu durchtrennen. Sollte ein Durchtrennen des Gewebes nur mit dem Draht nicht oder nur unzureichend möglich sein, so kann der Draht mittels entsprechender Stromversorgung mit geeigneter HF-Wechselspannung beaufschlagt werden, um im Wege einer koagulativen Gewebedurchtrennung eine gewünschte Steigerung des Durchlässigkeitsvermögens für das Kammerwasser innerhalb des Trabekelwerkes zu erzielen.

Diese Methode der mechanischen lokalen Durchtrennung des Trabekelwerkes mit Hilfe einer Drahtschlinge ist besonders deshalb vorteilhaft, zumal die Ausbildung der Drahtschlinge zu einer Eigenstabilität führt, die von einem losen Drahtende, das man möglicherweise zu ähnlichen Zwecken verwendet, nicht erreicht werden kann. Auf diese Weise wird auch die Operationssicherheit erheblich erhöht.

Eine weitere alternative Ausbildung sieht den Stents mit nur einer kleineren, zusätzlichen Öffnung, die mittig relativ zur Ablußöffnung platziert ist, vor. Der Stent selbst wird zur Handhabung, vorzugsweise im Bereich seiner Abflußöffnung mit einer Drahtschlinge umwickelt, wobei die zwei losen Drahtenden von einem Halte- und Betätigungsinstrument gehalten werden, um auf diese Weise den Stent kontrolliert in den Schlemm'schen grob zu platzieren. Um den Stent innerhalb des Schlemm'schen Kanals exakt zu zentrieren, ist ein Zentrierdorn vorgesehen, der in die kleinere Öffnung einführbar ist und auf diese Weise zur Feinjustierung dient.

Ist der Stent innerhalb des Schlemm'schen Kanals zentriert, so kann die Drahtschlinge gelockert werden und kann zudem als Schneidewerkzeug dienen um das an dem Stent anliegende Trabekelwerk zumindest teilsweise zu durchtrennen. Auch ist es möglich an dem Draht eine HF-Spannungn anzuelegen, die zum verbesserten Durchtrennen des Gewebes im Trabekelwerk beiträgt.

### Kurze Beschreibung der Zeichnungen

Die Erfindung wird nachstehend ohne Beschränkung des allgemeinen Erfindungsgedankens anhand von Ausführungsbeispielen unter Bezugnahme auf die Zeichnungen exemplarisch beschrieben. Es zeigen:
- Fig. 1 a, b, c: Dreiseitendarstellung eines erfindungsgemäß ausgebildeten röhrchenförmigen Elements
- Fig. 2 a, b: Darstellungen zum Einbringen des röhrchenförmigen Elements in den Schlemm'schen Kanal und
- Fig. 3 a, b: Erfindungsgemäß ausgebildetes röhrchenförmiges Element mit Haltedraht-Öffnungen.

### Beschreibung von Ausführungsbeispielen und gewerblicher Anwendbarkeit

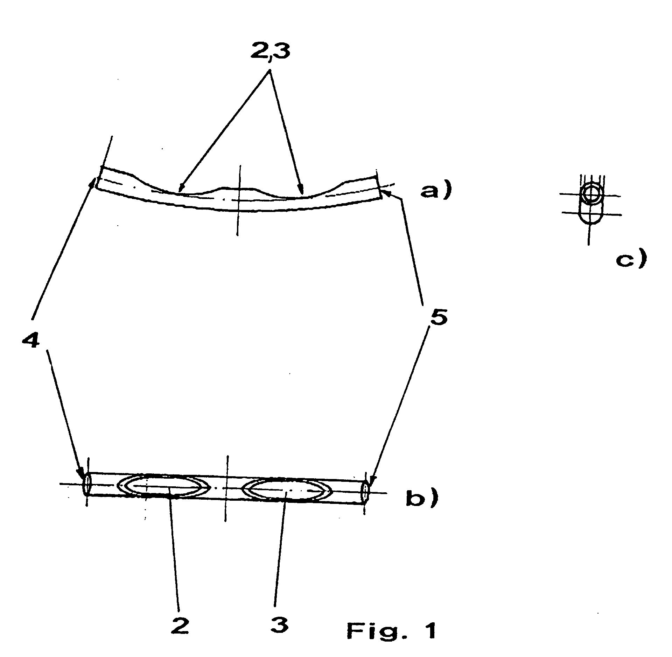

In Figur 1 a ist eine Seitendarstellung durch das röhrchenförmige Element 1 gezeigt. Es weist lang seiner Längserstreckung eine Krümmung auf. Konkavseitig sind zwei Öffnungen 2, 3 in die Außenwandung des röhrchenförmigen Elements eingearbeitet. Beidseitig an den Endbereichen des röhrchenförmigen Elements sind zwei Öffnungen 4, 5, vorgesehen.

Alle Kanten der Öffnungen 2, 3, 4 und 5 sind feinstpoliert und abgerundet um traumatische Gewebeirritationen zu vermeiden.

Der Innendurchmesser des in der Figur 1 c dargestellten Querschnitts beträgt im gezeigten Fall 170 µm, der Außendurchmesser 270 µm.

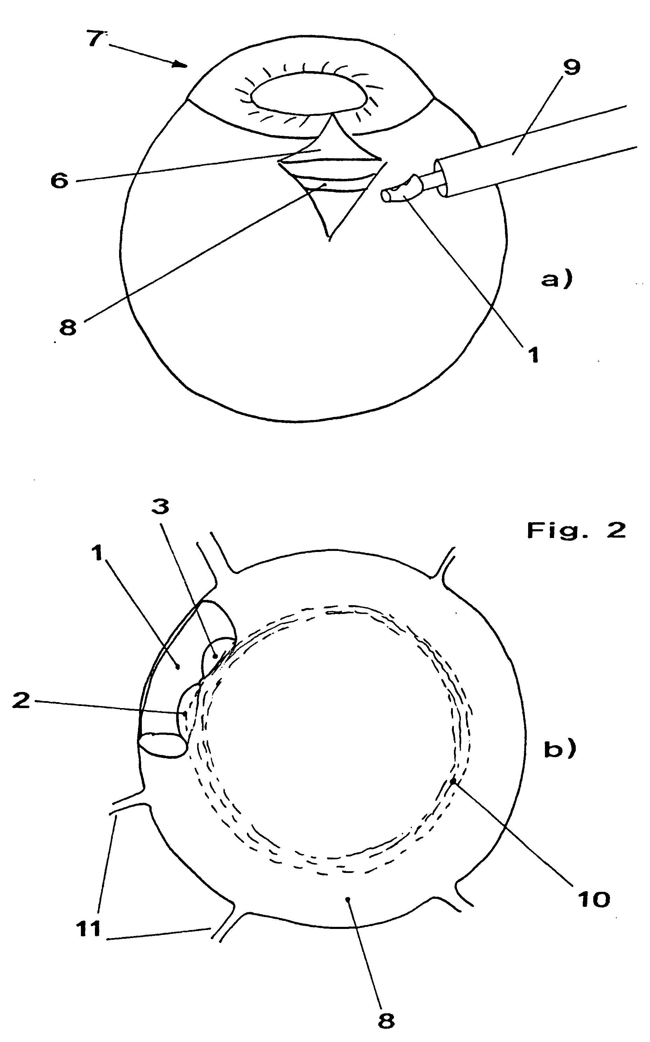

Durch eine geeignete Operationstechnik, die im wesentlichen die vom Erfinder beschriebene Modifikation der Trabekulotomie ist, wird unter einer Skleralamelle, der Lederhaut 6 (siehe Figur 2 a) am Auge7 der Schlemm'sche Kanal von außen aufgesucht und eröffnet. Das Röhrchen 1 (Glaukomstent) wird in den Schlemm'schen Kanal 8 eingebracht und je nach Bedarf an eine geeignete Stelle plaziert. Hierfür können sich speziell angefertigte Einführungsinstrumente 9 als dienlich erweisen. Der Schlemm'sche Kanals 8 wird sodann wieder verschlossen und die Skleralamelle wieder in ihrem Bett fixiert, was entweder durch Nähte oder durch Gewebekleber geschehen kann.

Die Senkung des Augeninnendruckes kann nun entweder allein dadurch erfolgen, daß das Trabekelwerk 10 (siehe Figur 2 b) über den Öffnungen 2, 3 des Glaukomstents 1 ausgespannt ist und das Maschenwerk 10 dadurch soweit erweitert ist, daß in diesem Bereich eine für die Drucksenkung genügende Absenkung des Abflußwiderstandes zustandekommt.

Ist dies nicht der Fall, wird das Trabekelwerk 10 über eine oder mehrere, an der konkaven Seite des Stents befindlichen Öffnungen oder über eine der seitlichen Öffnungen des Stents eröffnet, was entweder intraoperativ chirurgisch oder postoperativ beispielsweise durch disruptive Laserapplikation, aber auch andere denkbare Verfahren erfolgen kann.

Das Kammerwasser gewinnt nun durch diese neugeschaffenen Öffnungen Zugang zum Stent und über dessen beide seitlichen Öffnungen 4, 5 die im intakten Schlemm'schen Kanal 8 stecken, Zugang zur gesamten Circumferenz des Kanals und den daraus ausmündenden natürlichen Kammerwasser-Abflußwegen 11. Eine die Öffnung verschließende Wundheilung kann nicht erfolgen, weil ein Kollabieren der Öffnungsgrenzen durch den Glaukomstent und der Zugang zum Schlemm'schen Kanal durch ihn aufgehalten wird.

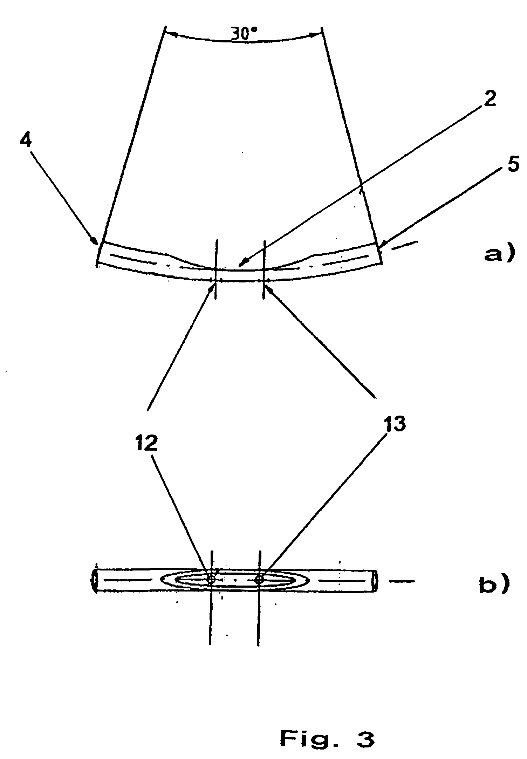

In den Figuren 3 a und b ist ein weiterführendes Ausführungsbeispiel eines Stents dargestellt, das zusätzlich zu dem Ausführungsbeispiel gemäß Figur 1 zwei weitere kleine Öffnungen 12 und 13 vorsieht, die auf der Außenseite des Stents, also der der Öffnung 2 gegenüberliegenden Öffnung, angebracht sind. Im Gegensatz zur Figur 1 sieht der Stent in Figur 3 nur eine Öffnung 2 auf, der dem Trabekelwerk zugewandten Seite auf. Die kleinen Öffnungen 12, 13 sind in Stentlängsrichtung nebeneinanderliegend (Fig. 3b) angeordnet und weisen je einen Durchmesser auf, der in der Größenordnung von einem Millimeter und darunter liegt. Durch die Öffnungen ist ein, nicht in der Figur dargestellter Draht, vorzugsweise den elektrischen Strom gut leitender Metalldraht, zu stecken, der derart durch die Öffnungen 12 und 13 geführt ist, so daß der Draht auf der konkav gekrümmten Seite des Stent eine Schlinge bildet. Die beiden losen Enden des Drahtes münden durch die Öffnungen 12 und 13 auf der konvex gekrümmten Seite, auf der sie von einem entsprechenden Halte- und Betätigungsinstrument (nicht dargesetllt) gefaßt sind.

Mit dem Halte- und Betätigungsinstrument kann der Stent sicher in den Schlemm'schen Kanal eingebracht und dort zum festen Verbleib positioniert werden.

Ferner ist es möglich mit Hilfe des Halte- und Betätigungsinstruments die Schlinge des Drahtes in den Gewebebereich des Trabekelwerkes zu schieben, um dort das Gewebe lokal zu durchtrennen und auf diese Weise die Durchlässigkeit des Trabekelwerkes für das Kammerwasser, insbesondere in unmittelbarer Nähe zur Öffnung des Stents, zu erhöhen. Auch ist es möglich den Draht mit Hochspannung zu versorgen, um den Durchtrennungsvorgang innerhalb des Gewebes zu optimieren. So kann das Gewebe mit einem durch Hochspannung erhitzten Draht regelrecht koagulativ gezielt durchtrennt bzw. perforriert werden. Auch ist es möglich den u.U. mit HF-Spannung beaufschlagten Draht einseitig durch die Öffung in das Trabekelwerk zu schieben, um auf diese Weise eine Perforierung dieses Gewebebereiches herbeizuführen.

### Bezugszeichenliste

- 1: röhrchenartig ausgebildetes Element, Stent
- 2, 3: Öffnung in Wandung des Stents, dem Trabekelwerk zugewandt
- 4, 5: Öffnungen in Längserstreckung des Stents
- 6: Lederhaut
- 7: Auge
- 8: Schlemm'scher Kanal
- 9: Einführinstrument
- 10: Trabekelwerk
- 11: Kammerwasser-Abflußweg
- 12,13: kleine Öffnungen

## Patentansprüche

1. Vorrichtung zur gezielten Verbesserung und/oder dauerhaften Gewährleistung des Durchlässigkeitsvermögens für Augenkammerwasser durch das Trabekelwerk (10) in den Schlemm'schen Kanal (8), mit einem röhrchenartig, mit kreisrunden Querschnitt ausgebildeten Element (1), dessen Wandmaterial einen Hohlkanal einschließt, der beidseitig in Längserstreckung des Hohlkanals offen ausgebildet ist und gekrümmt geformt ist und an seiner konkav gekrümmten Seite wenigstens eine Öffnung (2,3) aufweist, daß Größe und Form des röhrchenartig ausgebildeten Elements (1) in etwa der Innenkontur des Schlemm'schen Kanals (8) entspricht, und daß das Wandmaterial sowie die Wandstärke derart gewählt sind, daß das röhrchenartig ausgebildete Element (1) nach Einbringen in den Schlemm'schen Kanal (8) diesen sowie das angrenzende Trabekelwerk (10) aufspannt und innerhalb des Schlemm'schen Kanal (8) plaziert verbleibt,
**dadurch gekennzeichnet, dass** die Öffnung (2, 3) eine kleinste Öffnungsbreite aufweist, die dem Innendurchmesser des röhrchenartig ausgebildeten Elements (1) entspricht, der zwischen 150 µm und 200 µm misst.

2. Vorrichtung nach Anspruch 1,
**dadurch gekennzeichnet, daß** das Wandmaterial aus einem Geflecht gearbeitet ist und über die gesamte Längserstreckung durchlässig für das Kammerwasser ist.

3. Vorrichtung nach Anspruch 1 oder 2,
**dadurch gekennzeichnet, daß** das Wandmaterial aus Titan, galvanisch vergoldetem Implantationsstahl oder aus einem Kunststoff-Verbundwerkstoff.

4. Vorrichtung nach einem der Ansprüche 1 bis 3,
**dadurch gekennzeichnet, daß** das röhrchenartig ausgebildete Element (1) einen Außendurchmesser im Bereich zwischen 180 µm und 350 µm aufweist.

5. Vorrichtung nach einem der Ansprüche 1 bis 4,
**dadurch gekennzeichnet, daß** jegliche Kanten feinstpoliert und abgerundet sind.

6. Vorrichtung nach einem der Ansprüche 1 bis 5,
**dadurch gekennzeichnet, daß** die Länge des röhrchenartig ausgebildeten Elements (1) 1 mm bis 11 mm bzw. 30° bis 90° Bogenmaß des ringförmig ausgebildeten Schlemm'schen Kanals (8) beträgt.

7. Vorrichtung nach einem der Ansprüche 1 bis 6,
**dadurch gekennzeichnet, daß** das röhrchenartig ausgebildete Element (1) an seiner konvex gekrümmten Seite wenigstens eine kleinere Öffnung (12, 13) vorsieht, die in Projektion innerhalb der, auf der konkav gekrümmten Seite des röhrchenartig ausgebildeten Elements (1) befindlichen Öffnung (2, 3) liegt.

## Claims

1. A device for targeted improvement and/or permanently ensuring the permeability for ocular aqueous humour through the trabecular meshwork (10) into Schlemm's canal (8), with a tubular element (1) having a circular cross-section, whereof the wall material includes a hollow duct, which is designed open on both sides in longitudinal extension of the hollow duct, and has at least one opening (2, 3) on its concave curved side, in that size and form of the tubular element (1) correspond approximately to the inner contour of Schlemm's canal (8), and in that the wall material and the wall thickness are selected such that after it is inserted into Schlemm's canal (8) the tubular element (1) stenters the latter and the adjoining trabecular meshwork (10) and remains inside the Schlemm's canal (8), **characterised in that** the opening (2, 3) has the smallest opening width, which corresponds to the inner diameter of the tubular element (1), measuring between 150 µm and 200 µm.

2. The device as claimed in Claim 1, **characterised in that** the wall material is made of a weave and is permeable for the aqueous humor over the entire longitudinal extension.

3. The device as claimed in Claim 1 or 2, **characterised in that** the wall material comprises a solid material, preferably titanium, galvanically gilded implantation steel or a plastic bonded material.

4. The device as claimed in any one of Claims 1 to 3, **characterised in that** the tubular element (1) has an external diameter in the region of between 180 µm and 350 µm.

5. The device as claimed in any one of Claims 1 to 4, **characterised in that** any edges are fine-polished and rounded.

6. The device as claimed in any one of Claims 1 to 5, **characterised in that** the length of the tubular element (1) is approximately 1 to 11 mm or respectively 30° to 90° radian vestibular of the annular Schlemm's canal (8).

7. The device as claimed in any one of Claims 1 to 6, **characterised in that** on its convex side the tubular element (1) has at least one smaller opening (12, 13), which lies in projection inside the opening (2, 3) situated on the concave side of the tubular element (1).

## Revendications

1. Dispositif pour l'amélioration ciblée et/ou la garantie durable d'une capacité de perméabilité pour l'humeur aqueuse de l'oeil à travers le diaphragme (10) dans le canal lacrymal (8) avec un élément en forme de petit tube, de section circulaire (1), dont le matériau de paroi englobe un canal creux qui présente une conformation ouverte des deux côtés dans le développement longitudinal du canal creux, est courbe et présente sur sa face concave au moins une ouverture (2, 3) la taille et la forme de l'élément en forme de petit tube correspondant approximativement au contour interne du canal lacrymal (8), et le matériau ainsi que l'épaisseur de paroi étant choisies de telle sorte que l'élément en forme de petit tube soit sous tension après son introduction dans le canal lacrymal, ainsi que par ailleurs le diaphragme (10), et qu'il reste en position à l'intérieur du canal lacrymal (8), **caractérisé en ce que** l'ouverture (2, 3) présente une très petite largeur d'ouverture, qui correspond au diamètre intérieur de l'élément (1) en forme de petit tube, qui mesure entre 150µm et 200µm.

2. Dispositif selon la revendication 1, **caractérisé en ce que** le matériau de paroi est élaboré à partir d'une tresse et est perméable à l'humeur aqueuse sur l'ensemble de son développement longitudinal.

3. Dispositif selon la revendication 1 ou 2, **caractérisé en ce que** le matériau de paroi se compose de titane, d'acier pour implant à dorure galvanique ou d'un mélange plastique-composite.

4. Dispositif selon l'une des revendications 1 à 3, **caractérisé en ce que** l'élément en forme de petit tube (1) présente un diamètre extérieur compris entre 180µm et 350µm.

5. Dispositif selon l'une des revendications 1 à 4, **caractérisé en ce que** des arêtes associées sont finement polies et arrondies.

6. Dispositif selon l'une des revendications 1 à 5, **caractérisé en ce que** la longueur de l'élément en forme de petit tube (1) est de l'ordre de 1 à 11 mm ou de 30° à 90° de la longueur de l'arc du canal lacrymal de forme annulaire (8).

7. Dispositif selon l'une des revendications 1 à 6, **caractérisé en ce que** l'élément en forme de petit tube (1) présente sur sa face convexe au moins une petite ouverture (12, 13), qui est située en projection à l'intérieur de l'ouverture (2, 3) qui se trouve sur la face concave de l'élément en forme de petit tube (1).
